# EUROPEAN PATENT SPECIFICATION

(11) **EP 1 845 966 B1**
(45) Date of publication and mention of the grant of the patent: **21.07.2010**
(21) Application number: 06700207.1
(22) Date of filing: 10.01.2006
(51) Int. Cl.: A61K 31/194, A61P 35/00

(54) **PHARMACEUTICAL PREPARATION FOR USE IN THE TREATMENT OR PROPHYLAXIS OF NEOPLASTIC DISEASES**
PHARMAZEUTISCHE ZUBEREITUNG ZUR VERWENDUNG IN DER THERAPIE ODER PROPHYLAXE VON NEOPLASTISCHEN ERKRANKUNGEN
PREPARATION PHARMACEUTIQUE POUR SON UTILISATION DANS LA THÉRAPIE OU PROPHYLAXE DES MALADIES NEOPLASTIQUES

(30) Priority: 11.01.2005 PL 37218305
(43) Date of publication of application: 24.10.2007
(73) Proprietor: SGP & SONS AB, 211 15 Malmö (SE); Pierzynowski, Stefan G., 211 14 Malmö (SE); Kandefer-Szerszen, Martyna, 20-045 Lublin (PL); Rzeski, Wojciech, 20-830 Lublin (PL)
(72) Inventor: PYERZYNOWSKI, Stephan, 211 14 Malmö (SE); KANDEFER-SZERSZEN, Martyna, 20-045 Lublin (PL); RZESKI, Wojciech, 20-830 Lublin (PL)
(74) Representative: Bengtsson, Peggy Katrin
(86) International application number: PCT/PL2006/000003
(87) International publication number: WO 2006/075924

(56) References cited:
- WO-A-2004/012662
- WO-A-2006/066244
- US-A- 5 006 551
- US-A1- 2005 124 684
- MIZUSHINA Y; YAGITA E; KURAMOCHI K; ET AL: "5-(Hydroxymethyl)-2-furfural: A selective inhibitor of DNA polymerase lambda and terminal deoxynucleotidyltransferase" ARCHIVES OF BIOCHEMISTRY AND BIOPHYSICS, vol. 446, no. 1, 1 February 2006 (2006-02-01), pages 69-76, XP024942984
- MICHAIL K; JUAN H; MAIER A; MATZI V; GREILBERGER J; WINTERSTEIGER R: "Development and validation of a liquid chromatographic method for the determination of hydroxymethylfurfural and alpha-ketoglutaric acid in human plasma." ANALYTICA CHIMICA ACTA, vol. 581, no. 2, 26 August 2006 (2006-08-26), pages 287-297,
- MICHAIL K; MATZI V; MAIER A; HERWIG R; GREILBERGER J; JUAN H; KUNERT O; WINTERSTEIGER R: "Hydroxymethylfurfural: an enemy or a friendly xenobiotic? A bioanalytical approach" ANALYTICAL AND BIOANALYTICAL CHEMISTRY, vol. 387, no. 8, 14 February 2007 (2007-02-14), pages 2801-2814, XP019488806

## Description

An object of the invention is antineoplastic preparation and the use of antineoplastic preparation.

In spite of the significant progress in the chemotherapy, radiotherapy and immunotherapy in last years the problem of the effective antineoplastic therapy is still the serious challenge in a present medicine. Epidemiological research indicates that in countries fully developed one of three people suffers from the different neoplastic diseases. In that group each fourth case is a lethal one. Cytostatics actually used in treatment are at the risk of side effects occurence limiting the effectivness and decreasing the quality of patients life.

Therefore the development of new drugs acting specifically on the neoplastic cells with the simultaneous protective activity on the normal cells is especially urgent challenge.

US 5,006,551 discloses the use of alpha-ketoglutarate (AKG) in combination with hydroxymethylfurfural (HMF) as a composition having a destructive effect on malignant tumors. However, the US 5,006,551 document teaches that the HMF component is essential for the AKG to be effective.

Surprisingly, the inventor has shown that AKG is in fact effective as an antineoplastic active compound in isolation. As HMF has potential toxic, mutagenic, and carcinogenic effects, application of AKG without HMF is advantageous to circumvent problems arising from the said properties of HMF.

WO 2006/066244, which was published after the priority date of the present application, describes the effect of oxaloacetate on the life-span of *C. elegans* and *D. melanogaster.* From experimental results obtained with oxaloacetate, and from the metabolic connection between oxaloacetate and AKG, it is speculated that that AKG would have the same effects on the life-span of worms and flies as oxaloacetate. The authors of WO 2006/066244 go one step further and speculate on a potential antiproliferative activity of oxaloacetate, based on the fact that mammals under calorie restriction have a cancer rate which is reduced by up to 40%. This leads the authors to claim the use of not only oxaloacetate, but also of AKG for cancer therapy. No experimental data supporting is provided, and the use of metal salts of AKG for the treatment of cancer is not disclosed.

Thus, the invention provides a pharmaceutical preparation wherein the active component consists of mono- and/or divalent metal salts of alpha-ketoglutarate, for use in the treatment or prophylaxis of a neoplastic disease.

The invention also provides a pharmaceutical preparation for use as indicated above, wherein said treatment or prophylaxis of a neoplastic disease is inhibition of cancer metastases.

In other words, the invention provides the use of mono- and/or divalent metal salts of alpha-ketoglutarate in the manufacture of a pharmaceutical preparation for the treatment or prophylaxis of a neoplastic disease, wherein the active component of the pharmaceutical preparation consists of mono- and/or divalent metal salts of alpha-ketoglutarate. Also provided is use in the manufacture of a pharmaceutical preparation as stated above, wherein said treatment or prophylaxis of a neoplastic disease is inhibition of cancer metastases.

The preparation and the use of the preparation lets for the inhibition of the migration of neoplastic cells reflecting the potential role of AKG in the metastases inhibition. The preparation added to the diet plays a role as neo-adiuvant supporting existing methods used in the neoplasm treatment. It may improve the quality of the patients life through the synergic action with antineoplastic drugs and simultaneous protective activity to normal cells.

The example of the preparation action and its use is shown as a way of invention executing: Figure 1 - the curve presenting the proliferation of A549 cells with the stimulation by AKG, Figure 2 - the proliferation of C6 cells with AKG and Figure 3 - the proliferation of HT-29 cells with AKG stimulation, whereas Figure 4 presents the proliferation of human neoplastic cells A549 in the presence of the cyclophosphamide with AKG, while Figure 5 - the proliferation of human neoplastic cells A549 in the presence of iphosphamide with AKG, Figure 6 - the proliferation of human neoplastic cells A549 in the presence of thiotepa with AKG, Figure 7 - the inhibition of C6 cells migration due to AKG.

The cultures of neoplastic cells:
A549 - human neoplastic cells of the lung cancer; the continuous line obtained from the Institute of the Immunology and the Experimental Therapy of Polish Academy of Science in Wroclaw
HT-29 - human neoplastic cells of the large intestine cancer, the continuous line obtained from the Institute of the Immunology and the Experimental Therapy of Polish Academy of Science in Wroclaw
C6 - the rat neoplastic cells of the brain cancer (glioma); the continuous line obtained from the Department of Neonatology, Humboldt University, Berlin, Germany

### The bases of the culture.

Cells of A549 line were cultured on the basis DMEM:F-12 HAM (2:1), HT-29 and C6 cells on the basis DMEM. To the culture basis 10% foetal beast serum (FBS), penicillin 100 i.u./ml and streptomycin 100 µg/ml were added. The bases DMEM:F-12 HAM, DMEM were produced by Sigma company (Sigma, St. Louis, MO, U.S.A.). The foetal beast serum (FBS) was produced by Life Technologies company (Life Technologies, Karlsruhe, Germany). Remaining reagents were produced by Sigma company.

### The preparation of cellular cultures.

Cells stored in a liquid nitrogen in a tissue bank were unfreezed in a temp. 37°C, then poured into the plastic bottles containing proper basis. They were cultured in a temperature 37°C in the incubator with 5% CO2 flow. After cells reproduction liquid was poured out, cells were washed with PBS (without the calcium and magnesium ions) and processed with 0.25% trypsin solution + EDTA to receive the suspension of cells necessary in the experience.

### Assessment of the antyproliferative activity of AKG in the cellular culture.

Prepared earlier, in the culture basis, suspension of cells with a density 1x10⁴ cells/ml (A549), 4x10⁴ cells/ml (HT-29) and 0.5x10⁴ cells/ml (C6) were poured into the 96-pits microplate with the flat bottom (NUNC company, Roskilde, Denmark) in the volume 100 µl/pit. After sticking of cells (24 hours) the liquid was carefully pulled down and then different concentrations of AKG and examined cytostatics (cyclophosphamide, iphosphamid, thiotepa) in liquid with 10% FBS (100 µl/pit) were added. The cultures on plates were sleft for 96 hours incubation in temp. 37°C, in the atmosphere 95% air and 5% CO2. The antyproliferative activity of examined substances was assessed with the method MTT.

### The method MTT (according to kit "Cell proliferation kit III", Boehringer Manheim).

This method was worked out to determine the proliferation and vitality of cells in studies on the cytotoxic and antyproliferative substances. In metabolically active cells tetrazolic yellow salt MTT is reduced to formazane blue with the mitochondrial dehydrases. Formazane crystals, insoluble in water, accumulate in cells and for their dissolutions the use of organic detergent, breaking the membrane and simultaneously solvent the dye, is necessary. For this purpose the buffer SDS-HCl with pH 7.4 is used. The concentration of released dye is evaluated quantitatively in the reader for 96-pits plates at the wavelength 570 nm. The colour intensity is directly proportional to the quantity of alive cells.
MTT solution in PBS condition in concentration 5 mg/ml was added into each pit on plastic plate in a dose 15 µl/pit. Plates were incubated for 3 hours in temp. 37°C. Thereafter buffer SDS-HCl in a dose 100 µl/pit was added and plates were left in temp. 37°C all night. The results were evaluated next day with the use of E-max Reader (Molecular Devices Corporation, Menlo Park, CA, U.S.A.).

### Evaluation of the cells migration degree with the method "wound assay"

This method is for estimation the activity of substances affecting the modility of cells *in vitro.* It is used in research on wounds healing, angiogenesis and neoplastic metastases.

C6 cells (1 x 10⁶) suspended in the culture basis with the addtion of 10% serum (FBS) were poured onto the culture plates (NUNC, Roskilde, Denmark) with 4 cm diameter. Next day in the equal layer of cells the flaw (wound) was done with the ending of automatic pipette and unsticked cells were removed by twice-rinsing of plates with PBS solution. Then AKG (10 and 20mM) dissolved in the culture basis was added to the prepared culture. Plates were incubated for 24 hours in a temperature 37°C and in a humid atmosphere 95% air and 5% CO₂. Thereafter the cultures were coloured with May-Grünwald-Giemza method. Then the microscopic analysis was performed with the microscope Olympus BX51 (Olympus Optical CO., LTD, Tokyo, Japan) with the use of the software analySIS® (Soft Imaging System GmbH, Münster, Germany). The degree of cells migration was assessed in cytometry as the number of cells which populated the wound done earlier in the layer of cells. At least 50 chosen fields on 8 photographs were assessed.

### Results: The estimation of antiproliferative activity of AKG

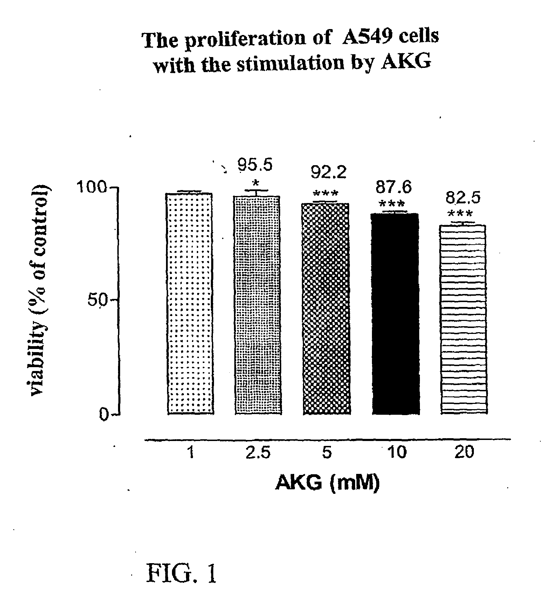

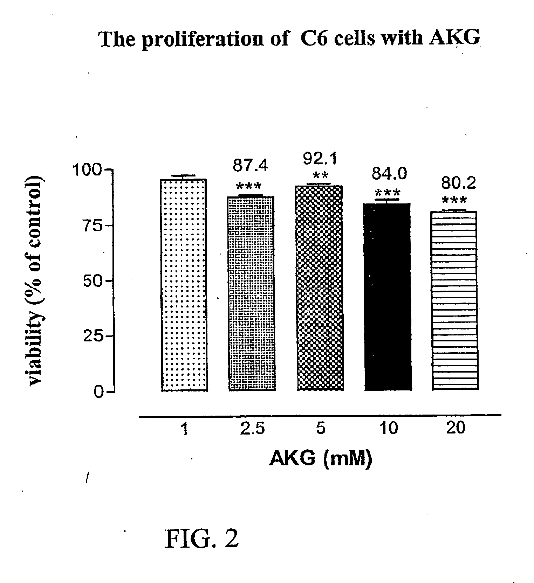

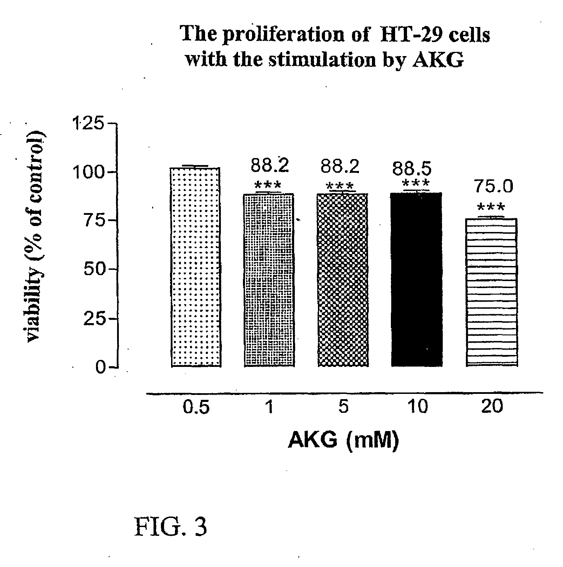

Antiproliferative activity of AKG in the cultures was estimated in different kind of neoplastic cells: the lung cancer cells (A549), the large intestine cancer cells (HT-29) and glioma cells (C6). Cells were processed with AKG in concentrations 0.5,1,2.5,5,10 and 20 mM, for 96 hours.
The examined substance has had antiproliferative activity with relation to all neoplastic cells types (the diagram - Fig.1, Fig.2, Fig.3). Statistically significant (4.5%) inhibition of cells growth was observed at AKG concentration 2.5mM in A549 cell line in the comparison to the control group. That effect was correlated with a dose of AKG and was 7.8%, 12.4%, 17.5% with doses 5mM, 10mM and 20mM respectively. (the diagram on Fig.1). The growth inhibition in glioma cells (C6) was 12.6% with AKG dose 2.5mM, 7.9% - 5mM, 16% - 10mM and 19.8%-20mM respectively. The growth inhibition of large intestine cancer cells (HT-29) hadn't had the lineal character with AKG concentrations between 1 and10mM. The dose 1mM inhidited the cells growth by 11.8%. The similar effect was obtained with a dose 5mM (11.8%) and 10mM (11.5%). Only a dose 20mM caused the significant (25%) inhibition of these cells growth (the diagram - Fig.3).

### The estimation of the interaction between AKG and antineoplastic drugs.

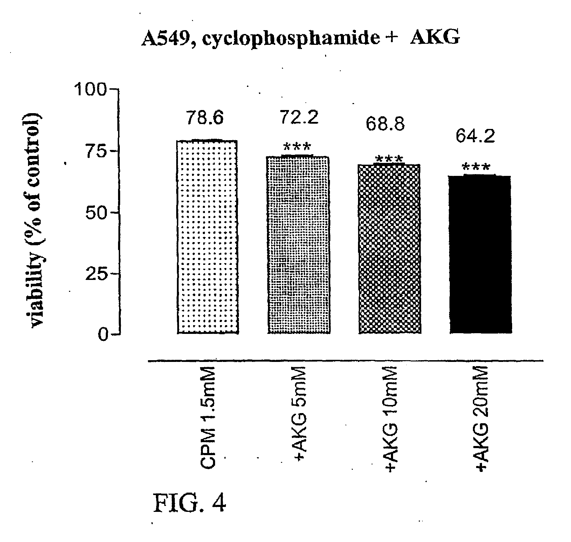

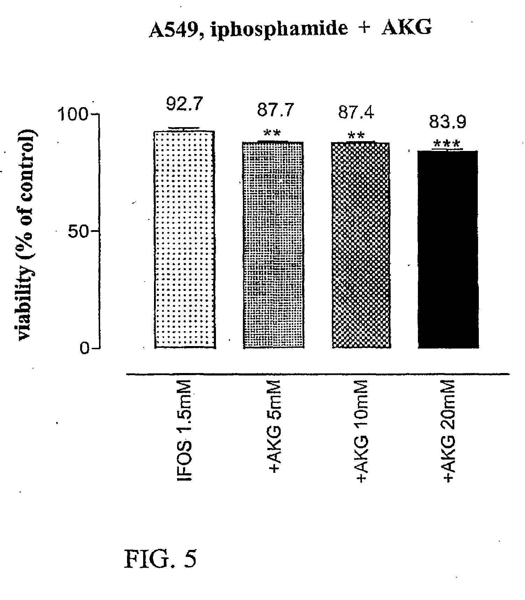

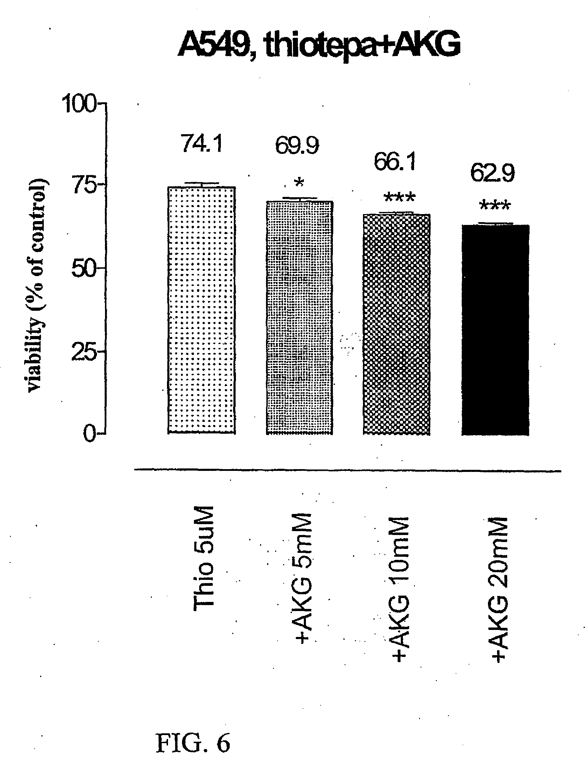

The research on the interaction between AKG and popular cytostatics used in cancer chemotherapy was performed in the culture of lung cancer cells (A549). For that purpose cells were treated with the following cytostatics: cyclophosphamide (1.5mM), iphosphamid (1.5mM) and thiotepa (5µM), alone and in combination with AKG (5, 10 and 20mM). The additive effect of AKG on cytostatic activity of used chemotherapeutics was observed (results are presented on Fig. 4, Fig.5 and Fig.6). Cyclophosphamide in a concentration 1.5mM inhidited the growth of A549 cells by 21.4%. The addition of AKG increased its cytostatic activity by 6.4%, 9.8% and 14.4% respectively (the diagram - Fig.4). Iphosphamid (1.5mM) inhidited the cells growth by 7.3%. After AKG addition (5, 10 and 20mM) that effect increased by 5%, 5.3% and 8.8% respectively (the diagram - Fig.5). AKG intensified also cytostatic activity of thiotepa (5µM - 25.9%) by 4.2%(5mM), 8% (10mM) and 11.2% (20mM) (results are presented on Fig.6).

### The influence of AKG on the migration of neoplastic cells

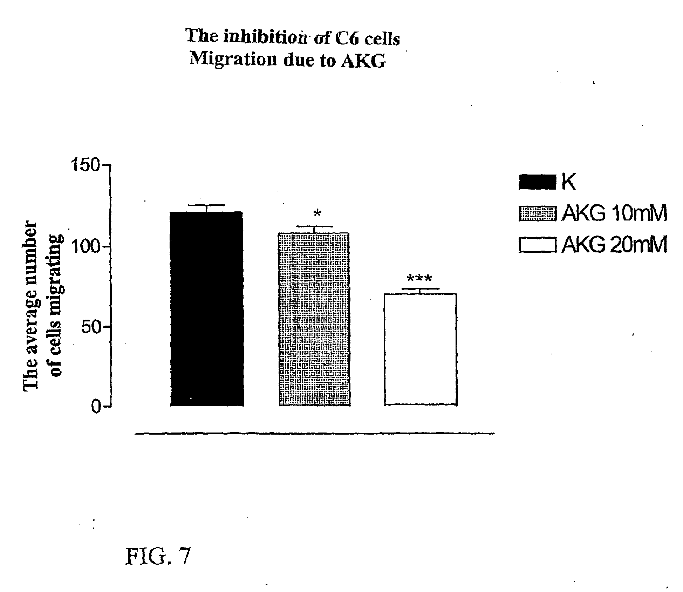

The research on the mobility of neoplastic cells was passed in the "wound assay" model. At the photograph 1 the wound in the layer of C6 cells (A), the wound population with cells after 24 hours of incubation without AKG (B) and significant inhibition of cells migration in the presence of AKG 20mM (C) are presented. On the diagram - Fig. 7, the average number of cells migrating to one field of flaw done in the uniform layer of cells is presented. Statistically significant inhibition of cells migration in the presence of 10 mM and 20 mM AKG was shown.

### The statistical analysis

The statistical analysis was performed with the use of t-student test. * p<0.05, ** p<0.01, ***p< 0.001.

## Claims

1. A pharmaceutical preparation wherein the active component consists of mono- and/or divalent metal salts of alpha-ketoglutarate, for use in the treatment or prophylaxis of a neoplastic disease.

2. A pharmaceutical preparation for use as claimed in claim 1, wherein said treatment or prophylaxis of a neoplastic disease is inhibition of cancer metastases.

## Patentansprüche

1. Pharmazeutische Zubereitung, worin der wirksame Bestandteile aus mono- und/oder divalenten Metallsalzen von α-Ketoglutarat besteht, zur Verwendung in der Behandlung oder Prophylaxe einer neoplastischen Erkrankung.

2. Pharmazeutische Zubereitung zur Verwendung, wie in Anspruch 1 beansprucht, wobei die Behandlung oder Prophylaxe einer neoplastischen Erkrankung eine Inhibition von Krebsmetastasen ist.

## Revendications

1. Préparation pharmaceutique dans laquelle le composant actif consiste en sels métalliques mono- et/ou divalents d'alpha-cétoglutarate, destinée à une utilisation pour le traitement ou la prophylaxie d'une maladie néoplasique.

2. Préparation pharmaceutique destinée à une utilisation selon la revendication 1, où ledit traitement ou ladite prophylaxie d'une maladie néoplasique est l'inhibition de métastases cancéreuses.
